(19) **Europäisches Patentamt**
**European Patent Office**
**Office européen des brevets**

(11) **EP 1 658 258 B1**

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **04740696.2**

(22) Anmeldetag: **06.07.2004**

(51) Int Cl.:
**C07C 51/215** (2006.01)

(86) Internationale Anmeldenummer:
**PCT/EP2004/007371**

(87) Internationale Veröffentlichungsnummer:
**WO 2005/019148 (03.03.2005 Gazette 2005/09)**

(54) **VERFAHREN ZUR HERSTELLUNG VON MALEINS UREANHYDRID**

METHOD FOR THE PRODUCTION OF MALEIC ANHYDRIDE

PROCEDE POUR PRODUIRE DE L'ANHYDRIDE D'ACIDE MALEIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.07.2003 DE 10334582**

(43) Veröffentlichungstag der Anmeldung:
**24.05.2006 Patentblatt 2006/21**

(73) Patentinhaber: **BASF SE**
**67056 Ludwigshafen (DE)**

(72) Erfinder:
• **DUDA, Mark**
**67071 Ludwigshafen (DE)**
• **MACHHAMMER, Otto**
**68163 Mannheim (DE)**
• **WECK, Alexander**
**67251 Freinsheim (DE)**

(56) Entgegenhaltungen:
**EP-A- 1 004 567** **US-A- 4 231 943**
**US-A- 4 342 699** **US-A- 6 040 460**

**Beschreibung**

Die vorliegende Erfindung betrifft ein Verfahren zur Herstellung von Maleinsäureanhydrid durch heterogenkatalytische Gasphasenoxidation von n-Butan mit Sauerstoff enthaltenden Gasen an einem Vanadium, Phosphor und Sauerstoff enthaltenden Katalysator in einer Reaktor-Einheit bei einer Temperatur im Bereich von 350 bis 500˚C, Abtrennung des gebildeten Maleinsäureanhydrids unter Bildung eines Gasstroms, welcher nicht-umgesetztes n-Butan und Wasser enthält und Rückführung von mindestens einem Teil des nicht-umgesetzten n-Butans zur Reaktor-Einheit.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Die Herstellung von Maleinsäureanhydrid durch heterogenkatalytische Gasphasenoxidation von Kohlenwasserstoffen mit mindestens vier Kohlenstoffatomen mit Sauerstoff an einem Vanadium-, Phosphor- und Sauerstoff-enthaltendem Katalysator ist allgemein bekannt und beispielsweise in Ullmann's Encyclopedia of Industrial Chemistry, 6th edition, 1999 Electronic Release, Chapter "MALEIC AND FUMARIC ACID - Maleic Anhydride" beschrieben. Im Allgemeinen werden Benzol oder $C_4$-Kohlenwasserstoffe, wie 1,3-Butadien, n-Butene oder n-Butan und insbesondere n-Butan eingesetzt. Die Reaktion ist stark exotherm und bedarf einer ausreichenden Abfuhr der Reaktionswärme. Im Allgemeinen führt man die Umsetzung in einem Rohrbündelreaktor mit Salzkreislauf oder einem Wirbelbett durch. Das bei der Umsetzung gebildete Maleinsäureanhydrid wird üblicherweise abgetrennt, wobei ein Abgasstrom, welcher unter anderem den nicht-umgesetzten Einsatz-Kohlenwasserstoff enthält, übrigbleibt. In der sogenannten "Single-Pass-Fahrweise" wird dieser Abgasstrom entsorgt, wohingegen in der sogenannten "Recycle-Pass-Fahrweise" der nicht-umgesetzte Einsatz-Kohlenwasserstoff zumindest teilweise wieder zum Reaktor zurückgeführt wird. Üblicherweise wird hierzu entweder der Einsatz-Kohlenwasserstoff abgetrennt, beispielsweise durch Absorption, und anschließend zum Reaktor zurückgeführt oder direkt ein Teil des Abgasstroms zum Reaktor zurückgeführt. Die "Recycle-Pass-Fahrweise" ermöglicht somit einen höheren Gesamt-Umsatz und daher eine bessere Nutzung des Einsatz-Kohlenwasserstoffs.

Die im Folgenden beschriebenen Verfahren aus der Patentliteratur betreffen die Herstellung von Maleinsäureanhydrid durch Umsetzung von n-Butan mit einem Sauerstoff enthaltenden Gas an einem Vanadium, Phosphor und Sauerstoff enthaltenden Katalysator in einer Reaktionszone in der "Recyde-Pass-Fahrweise", bei dem mindestens ein Teil des nach der Abtrennung des gebildeten Maleinsäureanhydrids erhaltenen Abgases zur Reaktionszone zurückgeführt wird beziehungsweise aus dem Abgasstrom n-Butan abgetrennt und zur Reaktionszone zurückgeführt wird.

US 5,532,284, US 5,646,304, US 5,726,327 und US 6,002,019 offenbaren allgemeine Verfahren zur Herstellung von cyclischen Anhydriden wie unter anderem Maleinsäureanhydrid durch heterogenkatalytische Oxidation eines geeigneten Kohlenwasserstoffs mit Sauerstoff, bei dem das cyciische Anhydrid aus dem Reaktor-Ausgangsstrom abgetrennt, das verbleibende Abgas zur Adsorption des nicht-umgesetzten Kohlenwasserstoffs einem Adsorber-Bett zugeführt und der adsorbierte Kohlenwasserstoff anschließend wieder desorbiert und zur Reaktionszone zurückgeführt wird.

Nachteilig an den in US 5,532,284, US 5,646,304, US 5,726,327 und US 6,002,019 offenbarten Verfahren ist der zwingende Einsatz einer Adsorptions-/Desorptionsstufe zur Abtrennung und Rückführung des nicht umgesetzten Kohlenwasserstoffs. Diese Adsorptions-/Desorptionsstufe ist ein weiterer Verfahrensschritt und erfordert entsprechende Investitionskosten und laufende Verfahrenskosten.

US 3,904,652 offenbart ein Verfahren, bei dem im Reaktor-Eingangsstrom die n-Butan-Konzentration mehr als 1,7 Vol.-%, die Sauerstoff-Konzentration 3 bis 13 Vol.-% beträgt, als Sauerstoff enthaltendes Gas ein Gas mit mindestens 50% Sauerstoffgehalt, bevorzugt "reiner" Sauerstoff mit einer Reinheit von mindestens 99,5% eingesetzt wird und mindestens ein Teil des nach der Abtrennung des gebildeten Maleinsäureanhydrids erhaltenen Abgasstroms zur Reaktionszone zurückgeführt wird. Da der zugeführte Reaktor-Eingangsstrom aufgrund der niedrigen Sauerstoff-Konzentration von maximal 13 Vol.-% unabhängig von der n-Butan-Konzentration außerhalb des Explosionsbereichs liegt, können auch n-Butan-Konzentrationen deutlich oberhalb 1,7 Vol.-% eingesetzt werden. Von einer n-Butan-Konzentration unterhalb 1,7 Vol.-% wird abgeraten, da diese die Reaktionsgeschwindigkeit verlangsamt sowie eine größere Katalysatormenge und eine höhere Reaktionstemperatur erfordert. Da der Reaktionsdruck als nicht kritisch beschrieben ist, kann die Umsetzung bei Atmosphärendruck, darüber oder darunter durchgeführt werden.

EP-A 0 029 317 offenbart ein Verfahren, bei dem im Reaktor-Eingangsstrom die n-Butan-Konzentration 25 bis 60 Vol.-%, die Sauerstoff-Konzentration 20 bis 45 Vol.-% beträgt und aus dem nach der Abtrennung des gebildeten Maleinsäureanhydrids erhaltenen Abgasstroms n-Butan auskondensiert und zur Reaktionszone zurückgeführt wird. Aufgrund der hohen Sauerstoff-Konzentration ist die Zufuhr mindestens eines mit Sauerstoff angereicherten Gases erforderlich, was in der Praxis letztendlich den Einsatz von "reinem" Sauerstoff bedeutet. Als Reaktionsdruck sind 0,5 bis 10 bar abs (0,05 bis 1,0 MPa abs) und bevorzugt 1 bis 3 bar abs (0,1 bis 0,3 MPa abs) genannt.

US 5,011,945 offenbart ein Verfahren, bei dem im Reaktor-Eingangsstrom die n-Butan-Konzentration 4 bis 10 Mol-% (= Vol.-%), die Sauerstoff-Konzentration 10 bis 18 Mol-% (= Vol.-%) beträgt, pro Reaktordurchgang ein n-Butan-Umsatz von 20 bis 35 Mol-% erzielt wird und mindestens ein Teil des nach der Abtrennung des gebildeten Maleinsäureanhydrids erhaltenen Abgasstroms zur Reaktionszone zurückgeführt wird. Wesentlich zur Erzielung einer hohen Ausbeute ist bei dem genannten Verfahren der Einsatz von gereinigtem Sauerstoff als Sauerstoff enthaltendes Gas, insbesondere von "reinem", mindestens 95%-igem Sauerstoff. Der Reaktionsdruck wird als nicht kritisch bezeichnet und sollte aus praktischen Gründen im Bereich von 10 bis 70 psia (0,069 bis 0,48 MPa abs) liegen.

EP-A 0 099 431 offenbart ein Verfahren, bei dem im Reaktor-Eingangsstrom die n-Butan-Konzentration 2 bis 10 Mol-% (= Vol.-%), die Sauerstoff-Konzentration 8 bis 20 Mol-% (= Vol.-%) beträgt, als Sauerstoff enthaltendes Gas ein Gas mit mindestens 95% Sauerstoffgehalt, bevorzugt "reiner" Sauerstoff eingesetzt wird, pro Reaktordurchgang ein niedriger n-Butan-Umsatz erzielt wird und mindestens ein Teil des nach der Abtrennung des gebildeten Maleinsäureanhydrids erhaltenen Abgasstroms zur Reaktionszone zurückgeführt wird. Wesentlich zur Erzielung einer hohen Ausbeute ist bei dem genannten Verfahren der Einsatz von gereinigtem Sauerstoff als Sauerstoff enthaltendes Gas, insbesondere von "reinem" Sauerstoff. Der Reaktionsdruck wird als nicht kritisch bezeichnet und sollte aus praktischen Gründen im Bereich von 10 bis 70 psia (0,069 bis 0,48 MPa abs) liegen.

Nachteilig an den in US 3,904,652, EP-A 0 029 317, US 5,011,945 und EP-A 0 099 431 offenbarten Verfahren ist der Einsatz von einem mit Sauerstoff angereichertem Gas beziehungsweise von "reinem" Sauerstoff. Da das mit Sauerstoff angereicherte Gas in der Praxis ebenfalls durch Zugabe von "reinem" Sauerstoff hergestellt wird, ist also in allen genannten Fällen der Einsatz von aufwändig zugänglichem und somit teuerem "reinen" Sauerstoff. Des Weiteren ist bei dem in EP-A 0 029 317 offenbarten Verfahren die relativ hohe Konzentration an n-Butan von 25 bis 60 Vol.-% und an Sauerstoff von 20 bis 45 Vol.-% sicherheitstechnisch mit Nachteilen verbunden, da auch bei Störungen durch entsprechende apparative und regelungstechnische Maßnahmen ein Absinken der n-Butan-Konzentration in den explosionsfähigen Bereich unterbunden werden muß.

US 4,231,943 offenbart ein Verfahren, bei dem im Reaktor-Eingangsstrom die n-Butan-Konzentration 1 bis 5 Vol.-% und besonders bevorzugt 2 bis 3,5 Vol.%, die Sauerstoff-Konzentration 6 bis 12 Vol.-% beträgt, pro Reaktordurchgang ein n-Butan-Umsatz von lediglich 15 bis 28% erzielt wird und ein Teil des nach der Abtrennung des gebildeten Maleinsäureanhydrids erhaltenen Abgasstroms zur Reaktionszone zurückgeführt und aus dem anderen Teil des Abgasstroms n-Butan isoliert und ebenfalls zur Reaktionszone zurückgeführt wird. Als Sauerstoff enthaltendes Gas kann Luft eingesetzt werden. Als Reaktionsdruck sind 10 bis 1000 psig (0,169 bis 7,0 MPa abs) und besonders bevorzugt 25 bis 40 psig (0,27 bis 0,38 MPa abs) genannt.

Nachteilig an dem in US 4,231,943 offenbarten Verfahren ist der geringe n-Butan-Umsatz pro Reaktordurchgang von 15 bis 28%. Dieser führt zu einer relativ hohen Menge von n-Butan im Reaktor-Ausgangsstrom. Aufgrund des Einsatzes von Luft mit ihrem hohen Anteil an Fremdgasen, insbesondere an Stickstoff, ist es zur Vermeidung einer Aufpegelung dieser Fremdgase in der Anlage erforderlich, einen entsprechend hohen Anteil als sogenannten Purge-Strom auszuschleusen. Somit kann daher nur ein gewisser Anteil dieses Fremdgas-haltigen Abgases direkt zum Reaktor zurückgeführt werden. Um die Ausschleusung von n-Butan so gering wie möglich zu halten, wird daher bei dem beschriebenen Verfahren n-Butan aus dem verbleibenden Teil des Abgases isoliert und ebenfalls zum Reaktor zurückgeführt. Die Isolation des n-Butans, welche beispielsweise durch Absorption und anschließende Desorption erfolgt, erfordert somit einen weiteren Verfahrensschritt mit entsprechenden Investitionskosten und laufenden Verfahrenskosten.

EP-A 0 690 040 offenbart ein Verfahren, bei dem im Reaktor-Eingangsstrom die n-Butan-Konzentration 1,6 bis 3,0 Vol.%, die Sauerstoff-Konzentration 10 bis 18 Vol.-% beträgt und mindestens ein Teil des nach der Abtrennung des gebildeten Maleinsäureanhydrids und nach einer Wäsche mit Wasser zur Entfernung der wasserlöslichen organischen Verbindungen erhaltenen Abgasstroms zur Reaktionszone zurückgeführt wird. Als Sauerstoff enthaltendes Gas können Luft, mit Sauerstoff angereicherte Luft oder reiner Sauerstoff eingesetzt werden. Als Reaktor-Eingangsdruck sind 2,03 bis 6,08 bar (0,203 bis 0,608 MPa) genannt.

Nachteilig an dem in EP-A 0 690 040 offenbarten Verfahren ist dessen Durchführung an der Grenze zum explosionsfähigen Bereich beziehungsweise sogar innerhalb des explosionsfähigen Bereichs. Dies wird unter Hinzunahme des Explosionsdiagramms aus EP-A 0 099 431 (Fig. 1) deutlich erkennbar. So liegt beispielsweise der größte Teil des in EP-A 0 690 040 genannten Bereichs mit einer n-Butan-Konzentration von 1,6 bis 3,0 Vol.-% und die Sauerstoff-Konzentration von 10 bis 18 Vol.-% im explosionsfähigen Bereich. Lediglich durch den Einsatz einer Sauerstoff-Konzentration nahe am unteren Ende des genannten Bereichs (also nahe 10 Vol.-%) und/oder durch den Einsatz einer n-Butan-Konzentrabon am unteren Ende des genannten Bereichs (also nahe 1,6 Vol.-%) ist ein sicherer Bereich möglich. Wie aus der Tabelle auf Seite 3 der EP-A 0 690 040 jedoch hervorgeht, ist mit einer n-Butan-Konzentration von 2,1 Vol.-% und einer Sauerstoff-Konzentration von 12.2 Vol.-% geradezu eine Fahrweise innerhalb des explosionsfähigen Bereichs als bevorzugt genannt.

Die Aufgabe der vorliegenden Erfindung bestand darin, ein Verfahren zur Herstellung von Maleinsäureanhydrid durch heterogenkatalytische Gasphasenoxidation von n-Butan mit Sauerstoff zu entwickeln, welches in Bezug auf das Durchgehen der Reaktion sicherheitstechnisch unproblematisch ist, mit entsprechendem Sicherheitsabstand außerhalb des Explosionsbereichs betrieben wird, eine hohe Ausnutzung des eingesetzten n-Butans durch Rückführung von mindestens einem Teil des nicht-umgesetzten n-Butans ermöglicht, in einfacher Art und Weise durchzuführen ist und welches einen hohen n-Butan-Umsatz, eine hohe Selektivität zu sowie eine hohe Ausbeute an Maleinsäureanhydrid und daher eine hohe Raum-Zeit-Ausbeute ermöglicht.

Demgemäß wurde ein Verfahren zur Herstellung von Maleinsäureanhydrid durch heterogenkatalytische Gasphasenoxidation von n-Butan mit Sauerstoff enthaltenden Gasen an einem Vanadium, Phosphor und Sauerstoff enthaltenden Katalysator in einer Reaktor-Einheit bei einer Temperatur im Bereich von 350 bis 500°C, Abtrennung des gebildeten Maleinsäureanhydrids unter Bildung eines Gasstroms, weicher nicht-umgesetztes n-Butan und Wasser enthält und Rückführung von mindestens einem Teil des nicht-umgesetzten n-Butans zur Reaktor-Einheit gefunden, welches dadurch gekennzeichnet ist, dass man der Reaktor-Einheit einen Eingangsstrom mit einer n-Butan-Konzentration von 0,5 bis 1,5 Vol.-% und einer Sauerstoff-Konzentration von 5 bis 21 Vol.-% zuführt, am Eingang der Reaktor-Einheit einen Druck von 0,4 bis 2 MPa abs einstellt und pro Reaktordurchgang 40 bis 100% des n-Butans aus dem Eingangsstrom umsetzt.

Die n-Butan-Konzentration des der Reaktor-Einheit zugeführten Eingangsstroms beträgt 0,5 bis 1,5 Vol.-%, bevorzugt 0,8 bis 1,5 Vol.-%, besonders bevorzugt 1 bis 1,5 Vol.-% und ganz besonders bevorzugt 1 bis 1,3 Vol.-%.

Als n-Butan enthaltender Einsatzstoff können prinzipiell alle n-Butan-haltigen Gase und Flüssigkeiten wie beispielsweise reines n-Butan oder n-Butan enthaltende Gemische verschiedener Kohlenwasserstoffe wie etwa 1,3-Butadien, 1-Buten, 2-cis-Buten, 2-trans-Buten, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und/oder Benzol eingesetzt werden.

Bevorzugt beträgt der n-Butan-Anteil an der Gesamtmenge des zugeführten Kohlenwasserstoff $\geq$ 90% und besonders bevorzugt $\geq$ 95%. Das verwendete n-Butan stammt dabei bevorzugt aus Erdöl-Begleitgas, Erdgas, Steamcrackem oder FCC-Crackern.

Die Zugabe von n-Butan beziehungsweise des n-Butan-haltigen Gases erfolgt im Allgemeinen verhältnismengengeregelt, d.h. unter stetiger Vorgabe eines definierten Verhältnisses zum zugeführten Gesamtmengenstrom am Reaktoreingang und damit der Konzentration. Das n-Butan beziehungsweise das n-Butan-haltigen Gas kann in flüssiger oder gasförmiger Form dosiert werden. Bevorzugt ist die gasförmige Dosierung, da diese gegenüber der Dosierung in flüssiger Form mit anschließender Verdampfung des n-Butans im Gefahrenfalle aufgrund des um Größenordnungen kleineren Holdups das Gefährdungspotenzial deutlich verringert.

Die Sauerstoff Konzentration des der Reaktor-Einheit zugeführten Eingangsstroms beträgt 5 bis 21 Vol.%, bevorzugt 8 bis 20 Vol.%, besonders bevorzugt 8 bis 15 Vol.-% und ganz besonders bevorzugt 8 bis 13 Vol.-%.

Als Sauerstoff enthaltende Einsatzstoffe werden im Allgemeinen Sauerstoff enthaltende Gase, wie beispielsweise Luft, synthetische Luft, mit Sauerstoff angereicherte Gase oder auch sogenannter "reiner", d.h. zum Beispiel aus der Luftzerlegung stammender Sauerstoff eingesetzt. Besonders bevorzugt ist der Einsatz von Luft als Sauerstoff enthaltendes Gas.

Der zu einhundert Vol.-% fehlende Anteil setzt sich aus weiteren Gasen wie beispielsweise Stickstoff, Edelgasen, Kohlenmonoxid, Kohlendioxid, Alkane (z.B. Propan, i-Butan, Pentan), Alkene (z.B. Butene), Wasserdampf, oxygenierte Kohlenwasserstoffe (z.B. Methanol, Formaldehyd, Ameisensäure, Ethanol, Acetyaldehyd, Essigsäure, Propanol, Propionaldehyd, Propionsäure, Acrolein, Crotonaldehyd) und deren Mischungen zusammen. Bevorzugt wird zudem im Reaktor-Eingangsstrom ein Wassergehalt von 0 bis 15 Vol.-% und bevorzugt von 1 bis 8 Vol.-%, gegebenenfalls durch separate Zufuhr von Wasserdampf, eingestellt.

Der Druck am Eingang der Reaktor-Einheit beträgt 0,4 bis 2 MPa abs, bevorzugt 0,4 bis 1,5 MPa abs, besonders bevorzugt 0,5 bis 1,2 MPa abs und ganz besonders bevorzugt 0,6 bis 1 MPa abs. Unter dem Eingang der Reaktor-Einheit ist die Stelle in der Reaktor-Einheit zu verstehen, an der der zugeführte Eingangsstrom erstmals mit dem Vanadium, Phosphor und Sauerstoff enthaltenden Katalysator in Kontakt kommt. Der genannte Druck wird im Allgemeinen durch die Druckregelung am Reaktorausgang eingestellt.

Das erfindungsgemäße Verfahren wird bei einer Temperatur von 350 bis 500°C durchgeführt. Unter der genannten Temperatur ist, unabhängig von der Art des Reaktors, jeweils die mittlere Temperatur des Wärmeträgermediums zu verstehen. Bevorzugt wird das erfindungsgemäße Verfahren bei einer Temperatur von 380 bis 460°C und besonders bevorzugt 380 bis 440°C durchgeführt.

Der n-Butan-Umsatz pro Reaktordurchgang beträgt 40 bis 100%, bevorzugt 50 bis 95% und besonders bevorzugt 70 bis 95% des n-Butans aus dem Eingangsstrom.

Beim Erfindungsgemäßen Verfahren stellt man über die Menge des Eingangsstroms in der Reaktor-Einheit eine GHSV (gaseous hourly space velocity) von bevorzugt 2000 bis 10000 $h^{-1}$ und besonders bevorzugt 3000 bis 6000 $h^{-1}$, bezogen auf das auf 0°C und 0,1013 MPa abs normierte Volumen des zugeführten Eingangsstroms und bezogen auf das Schüttvolumen des Katalystors summiert über alle Reaktionszonen, ein. Der Parameter GHSV ist im Kapitel "Definitionen" definiert.

Die heterogenkatalytische Gasphasenoxidation von n-Butan mit Sauerstoff enthaltenden Gasen in Gegenwart einer flüchtigen Phosphorverbindung an einem Vanadium, Phosphor und Sauerstoff enthaltenden Katalysator erfolgt in einer sogenannten Reaktor-Einheit Unter dem Begriff Reaktor-Einheit ist eine Einheit aus mindestens einem Reaktor zu verstehen. Sind mehrerer einzelne Reaktoren (im Sinne von Reaktor-Apparaten) parallel-geschaltet, so sind diese als Äquivalent eines Reaktors zu verstehen und im Folgenden im Begriff Reaktor enthalten.

Als Reaktoren können beim erfindungsgemäßen Verfahren prinzipiell alle Reaktoren eingesetzt werden, welche für heterogenkatalytische Gasphasenoxidationen geeignet sind. Als geeignete Reaktoren seien insbesondere Wirbelbett-, Linde- (Vllickel-), Plattenbündel- und Rohrbündelreaktoren genannt.

Bei einer bevorzugten Variante des erfindungsgemäßen Verfahrens setzt man als Reaktor-Einheit eine Wirbelbettreaktor-Einheit ein. Diese umfasst in der Regel einen oder mehrere parallel geschaltete Wirbelbettreaktoren.

Bei einer anderen und besonders bevorzugten Variante des erfindungsgemäßen Verfahrens setzt man als Reaktor-Einheit eine Rohrbündelreaktor-Einheit ein. Ein Rohrbündelreaktor besteht wiederum aus mindestens einem Reaktorrohr, welches zur Beheizung und/oder Kühlung von einem Wärmeträgermedium umgeben ist. Im Allgemeinen enthalten die technisch eingesetzten Rohrbündelreaktoren wenige hundert bis mehrere zehntausend parallel-geschaltete Reaktorrohre.

Eine Rohrbündelreaktor-Einheit kann eine oder mehrere Vorheizzonen enthalten, welche das eintretende Gasgemisch aufheizen. Eine in einem Rohrbündelreaktor integrierte Vorheizzone kann beispielsweise durch mit Inertmaterial gefüllte Reaktorrohre, welche ebenfalls von Wärmeträgermedium umgeben sind, realisiert werden. Als Inertmaterial sind prinzipiell alle Formkörper geeignet, welche chemisch inert sind, d.h. keine heterogenkatalytische Reaktion induzieren oder katalysieren, und welche einen maximalen Druckverlust unterhalb des jeweiligen, maximal tolerierbaren, anlagenspezifischen Wert aufweisen. Geeignet sind beispielsweise oxidische Materialen, wie etwa Aluminiumoxid, Siliciumcarbid oder metallische Materialien, wie etwa Edelstahl. Als Formkörper seien beispielsweise Kugeln, Tabletten, Hohlzyiinder, Ringe, Triiobes, Tristars, Wagenräder, Extrudate oder regellos gebrochene Formkörper genannt.

Des Weiteren kann beim Einsatz einer Rohrbündelreaktor-Einheit das Katalysatorbett durch Einbauten, wie beispielsweise Federn im Rohr fixiert sein.

Besteht die Rohrbündelreaktor-Einheit aus mehreren Rohrbündelreaktoren, beispielsweise zwei, drei, vier oder mehr, so können diese beispielsweise parallelgeschaltet oder hintereinandergeschaltet vorliegen. Bei einer Hintereinanderschaltung von Rohrbündelreaktoren wird der Ausgangsstrom des einen Rohrbündelreaktors direkt in den Eingang des folgenden Rohrbündelreaktors geleitet. Es ist aber auch möglich, zwischen den beiden Rohrbündelreaktoren Masse und/oder Energie ab- und/oder zuzuführen. So kann beispielsweise ein Teil des Gasstroms oder eine Komponente davon entnommen oder ein weiterer Gasstrom zugeführt werden oder der vorhandene Gasstrom durch einen Wärmetauscher geleitet werden.

Üblicherweise sind in den vorgenannten Rohrbündelreaktoren die Reaktorrohre aus ferritischem Stahl gefertigt und weisen in typischer Weise eine Wanddicke von 1 bis 3 mm auf. Ihr Innendurchmesser beträgt in der Regel 20 bis 30 mm. Die Anzahl der Reaktorrohre je Rohrbündelreaktor liegt üblicherweise im Bereich zwischen 5000 und 35000, obgleich auch eine Anzahl über 35000 bei besonders großen Anlagen realisiert werden kann. Innerhalb des Reaktorkorpus sind die Reaktorrohre im Normalfall homogen verteilt angeordnet.

Als Wärmeträgermedien eignen sich insbesondere fluide Temperiermedien. Besonders günstig ist die Verwendung von Salzschmelzen, wie Kaliumnitrat, Kaliumnitrit, Natriumnitrat und/oder Natriumnitrit oder von niedrig schmelzenden Metallen wie Natrium sowie Legierungen verschiedener Metalle. Es kann aber auch Kesselspeisewasser zugeführt und Dampf generiert werden; dieser kann ggf. überhitzt werden oder sogar bei Drücken über 22 MPa abs als überkritischer Wasserdampf abgezogen werden.

Wird beim erfindungsgemäßen Verfahren eine Rohrbündelreaktor-Einheit eingesetzt, so umfasst diese mindestens eine und bevorzugt mindestens zwei durch ein Wärmeträgermedium gekühlten Reaktionszonen. Unter dem Begriff Reaktionszone ist ein Bereich innerhalb eines Rohrbündelreaktors zu verstehen, welcher einen Katalysator enthält und bei dem die Temperatur infolge des umgebenden Wärmeträgermediums bei Abwesenheit einer chemischen Reaktion auf einen einheitlichen Wert gehalten werden würde. Im Allgemeinen wird die Reaktionszone durch die lokale Ausdehnung des Wärmeträgerkreislaufs abgegrenzt. So umfasst beispielsweise ein Rohrbündelreaktor mit nur einem Wärmeträgerkreislauf auch nur eine Reaktionszone, welche konventionsgemäß als erste Reaktionszone bezeichnet wird. Besteht eine Rohrbündelrekator-Einheit beispielsweise aus einem Rohrbündelreaktor mit zwei getrennten, nachfolgenden Wärmeträgerkreisläufen, so umfasst dieser zwei Reaktionszonen, wobei die Nummerierung der Reaktionszonen entsprechend der Durchleitungsrichtung des Gases erfolgt.

Wird beim erfindungsgemäßen Verfahren eine Rohrbündelreaktor-Einheit eingesetzt, so ist es im Allgemeinen vorteilhaft in mindestens einer der Reaktionszonen eine, hinsichtlich der Aktivität, strukturierte Katalysatorschüttung einzusetzen. Diese weist üblicherweise in einem Bereich niedriger Temperatur und geringer Kohlenwasserstoff Konzentration eine hohe Aktivität und in einem Bereich, in dem durch das Zusammenwirken von Temperatur und der vorliegenden Kohlenwasserstoff-Konzentration ein übermäßiges Ansteigen der Reaktionsgeschwindigkeit und der Temperatur hervorrufen könnte, eine niedrige Aktivität auf.

Die Strukturierung der Katalysatorschüttung kann durch verschiedene Maßnahmen, gegebenenfalls in deren Kombination, erreicht werden. So ist es beispielsweise möglich, den Katalysator mit Inertmaterial, zum Beispiel mit Formkörpern aus Steatit, Aluminiumoxid, Siliciumcarbid oder einem anderen inerten Material, zu verdünnen. Ferner ist auch eine Aktivitätsstrukturierung durch den Einsatz unterschiedlich aktiver Katalysatoren möglich. Diese wiederum kann durch eine unterschiedliche Formgebung und/oder durch den Einsatz unterschiedlicher Aktivmassen erreicht werden.

Die beim erfindungsgemäßen Verfahren einsetzbaren Vanadium, Phosphor und Sauerstoff enthaltenden Katalysatoren umfassen als katalytisch aktive Masse eine sauerstoffhaltige Vanadium-Phosphor-Verbindung oder Gemische solcher Verbindungen. Geeignete Aktivmassen sind beispielsweise in den Patentschriften US 5,275,996, US 5,641,722, US 5,137,860, US 5,095,125, US 4,933,312, US 5,498,731, US 4,525,471, US 5,302,566 oder DE 34 29164 beschrieben.

Sie können des weiteren sogenannte Promotoren enthalten. Als geeignete Promotoren sind die Elemente der 1. bis 15. Gruppe des Periodensystems sowie deren Verbindungen genannt. Geeignete Promotoren sind beispielsweise in den Offenlegungsschriften WO 97/12674 und WO 95/26817 sowie in den Patenten US 5,137,860, US 5,296,436, US 5,158,923 und US 4,795,818 beschrieben. Bevorzugt werden als Promotoren Verbindungen der Elemente Kobald, Molybdän, Eisen, Zink, Hafnium, Zirkon, Lithium, Titan, Chrom, Mangan, Nickel, Kupfer, Bor, Silicium, Antimon, Zinn, Niob und Wismut, besonders bevorzugt Molybdän, Eisen, Zink, Antimon, Wismut, Lithium. Die promotierten Katalysatoren können einen oder mehrere Promotoren enthalten. Der Gehalt an Promotoren beträgt in Summe im fertigen Katalysator im Allgemeinen nicht mehr als etwa 5 Gew.-%, jeweils als Oxid gerechnet.

Bei der Herstellung der Katalysatoren können ferner auch sogenannte Hilfsmittel, wie etwa Tablettierhilfsmittel oder Porenbildner eingesetzt werden.

Die beim erfindungsgemäßen Verfahren einsetzbaren Katalysatoren können die Aktivmasse beispielsweise in reiner, unverdünnter Form als sogenannter "Vollkatalysator" oder verdünnt mit einem bevorzugt oxidischen Trägermaterial als sogenannter "Mischkatalysator" enthalten. Als geeignete Trägermaterialien für Mischkatalysatoren seien beispielsweise Aluminiumoxid, Siliciumdioxid, Alumosilikate, Zirkondioxid, Titandioxid oder Gemische davon genannt. Bevorzugt ist die Herstellung von Voll- und Mischkatalysatoren, besonders bevorzugt von Vollkatalysatoren.

Der beim erfindungsgemäßen Verfahren in einem Rohrbündelreaktor bevorzugt einzusetzende Katalysator weist Partikel mit einem gemittelten Durchmesser von mindestens 2 mm, bevorzugt mindestens 3 mm auf. Unter dem gemittelten Durchmesser eines Partikels ist der Mittelwert aus der kleinsten und der größten Abmessung zwischen zwei planparallelen Platten zu verstehen. Der beim erfindungsgemäßen Verfahren in einem Wirbelbettreaktor bevorzugt einzusetzende Katalysator weist Partikel mit einem gemittelten Durchmesser von 10 bis 500 $\mu$m, bevorzugt von 50 bis 200 $\mu$m und besonders bevorzugt 50 bis 150 $\mu$m auf.

Unter Partikeln sind sowohl regellos geformte Partikel als auch geometrisch geformte Partikel, sogenannte Formkörper, zu verstehen. Bevorzugt weist der beim erfindungsgemäßen Verfahren einzusetzende Katalysatorprecursor Formkörper auf. Als geeignete Formkörper seien beispielsweise genannt Tabletten, Zylinder, Hohlzylinder, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Besonders bevorzugt weist der beim erfindungsgemäßen Verfahren einsetzbare Katalysator Formkörper mit einer im Wesentlichen hohlzylinderförmigen Struktur auf. Unter einer im wesentlichen hohlzylinderförmigen Struktur ist eine Struktur zu verstehen, welche im wesentlichen einen Zylinder mit einer zwischen den beiden Deckelflächen hindurchgehenden Öffnung umfaßt. Der Zylinder ist charakterisiert durch zwei im Wesentlichen parallele Deckelflächen und einer Mantelfläche, wobei der Querschnitt des Zylinders, d.h. parallel zu den Deckelflächen, im wesentlichen von kreisförmiger Struktur ist. Der Querschnitt der hindurchgehenden Öffnung, d.h. parallel zu den Deckelflächen des Zylinders, ist im wesentlichen ebenfalls von kreisförmiger Struktur. Bevorzugt befindet sich die hindurchgehende Öffnung mittig zu den Deckelflächen, wobei andere räumliche Anordnungen damit nicht ausgeschlossen sind.

Der Begriff "im Wesentlichen" weißt darauf hin, dass Abweichungen von der Idealgeometrie, wie beispielsweise leichte Deformationen der kreisförmigen Struktur, nicht planparallel ausgerichtete Deckelflächen, abgeplatzte Ecken und Kanten, Oberflächenrauhigkeit oder Einkerbungen in der Mantelfläche, den Deckelflächen oder der Innenfläche der hindurchgehenden Bohrung beim Katalysatorprecursor mit umfasst sind. Im Rahmen der Genauigkeit der Tablettierkunst sind kreisförmige Deckelflächen, ein kreisförmiger Querschnitt der hindurchgehenden Bohrung, parallel ausgerichtete Deckelflächen und makroskopisch glatte Oberflächen bevorzugt.

Die im Wesentlichen hohlzylinderförmige Struktur kann beschrieben werden durch einen äußeren Durchmesser $d_1$, einer Höhe h als Abstand der beiden Deckelflächen und einem Durchmesser des inneren Lochs (hindurchgehende Öffnung) $d_2$. Der äußere Durchmesser $d_1$ des Katalysatorprecursors beträgt bevorzugt 3 bis 10 mm, besonders bevorzugt 4 bis 8 mm, ganz besonders bevorzugt 5 bis 7 mm. Die Höhe h beträgt bevorzugt 1 bis 10 mm, besonders bevorzugt 2 bis 6 mm, ganz besonders bevorzugt 2,5 bis 4,5 mm. Der Durchmesser der hindurchgehenden Öffnung $d_2$ beträgt bevorzugt 1 bis 8 mm, besonders bevorzugt 2 bis 6 mm, ganz besonders bevorzugt 2,5 bis 4 mm.

Die Katalysatorherstellung ist im Allgemeinen ein mehrstufiger Prozeß, bei dem man zunächst eine sogenannten Katalysatorprecursor herstellt und diesen anschließend durch Kalzinierung in die aktive Form überführt. Die beim erfindungsgemäßen Verfahren einsetzbaren Katalysatorprecursor können beispielsweise wie in den Schriften US 5,275,996, US 5,641,722, WO 97/12674, WO 01/68626, WO 01/68245, WO 02/22257, WO 02134387, DE-Az. 10211 449.8, DE-Az. 102 11 445.5, DE-Az. 102 11 447.1, DE-Az. 102 11 446.3 und DE-Az. 102 35 355.7 beschrieben hergestellt werden.

Die beim erfindungsgemäßen Verfahren bevorzugt einzusetzenden Katalysatoren weisen ein PhosphorNanadium-Atomverhälinis von 0,9 bis 1,5, besonders bevorzugt von 0,9 bis 1,2 und ganz besonders bevorzugt von 1,0 bis 1,1, eine mittlere Oxidationsstufe des Vanadiums von +3,9 bis +4,4 und besonders bevorzugt von 4,0 bis 4,3, eine BET-Oberfläche von 10 bis 50 m$^2$/g und besonders bevorzugt von 20 bis 40 m$^2$/g, ein Porenvolumen von 0,1 bis 0,5 ml/g und besonders bevorzugt von 0,2 bis 0,4 ml/g und eine Schüttdichte von 0,5 bis 1,5 kg/l und besonders bevorzugt 0,5 bis 1,0 kg/l auf.

Zur Gewährung einer langen Katalysatorstandzeit und weiterer Erhöhung von Umsatz, Selektivität, Ausbeute, Katalysator-Belastung und Raum/Zeit-Ausbeute führt man die heterogenkatalytische Gasphasenoxidation bevorzugt in Gegenwart einer flüchtigen Phosphorverbindung durch. Ihre Konzentration beträgt im Feed am Reaktoreingang bevorzugt ≥0,2 Volumen-ppm, d.h. ≥0,2·10$^{-6}$ Volumenanteile der flüchtigen Phosphorverbindungen bezogen auf das Gesamtvolumen des Gases am Reaktoreingang. Besonders bevorzugt ist ein Gehalt von 0,2 bis 20 Volumen-ppm und ganz besonders bevorzugt von 0,5 bis 10 Volumen-ppm. Als flüchtige Phosphorverbindungen sind all jene Phosphor-enthaltende Verbindungen zu verstehen, welche in der gewünschten Konzentration unter den Einsatzbedingungen gasförmig vorliegen. Als geeignete flüchtige Phosphorverbindungen sind beispielsweise Phosphine, Phosphorsäureester und die in US 3,474,041 beschriebenen Verbindungen genannt. Besonders bevorzugt ist Tri-(C$_1$- bis C$_4$-alkyl)-phosphat und ganz besonders bevorzugt Trimethylphosphat, Triethylphosphat und Tripropylphosphat, insbesondere Triethylphosphat.

Die Zugabe der flüchtigen Phosphorverbindung kann kontinuierlich oder diskontinuierlich erfolgen. Bevorzugt ist die kontinuierliche Zufuhr.

Der aus der Reaktor-Einheit abgeführte Gasstrom wird im Allgemeinen einer anschlie-ßenden Verfahrens-Einheit zur Abtrennung des gebildeten Maleinsäureanhydrids zugeführt. Die Abtrennung von Maleinsäureanhydrid kann beispielsweise durch Absorption in einem geeigneten Absorptionsmittel erfolgen. Geeignete Absorptionsmittel sind beispielsweise Wasser oder organische Lösungsmittel. Bei Absorption in Wasser wird Maleinsäureanhydrid zu Maleinsäure hydratisiert. Bevorzugt ist die Absorption in einem organischen Lösungsmittel. Geeignete organische Lösungsmittel sind beispielsweise die in WO 97/43242 genannten hochsiedenden Lösungsmittel, wie Trikresylphosphat, Dibutylmaleat, hochmolekulares Wachs, aromatische Kohlenwasserstoffe mit einem Siedepunkt über 140°C oder Di-C$_4$-C$_8$-alkylphthalate, wie etwa Dibutylphthalat. In den genannten Lösungsmitteln werden im Allgemeinen auch oxygenierte Kohlenwasserstoff-Nebenprodukte absorbiert. Die Absorption kann beispielsweise bei einer Temperatur von 60 bis 160°C und einem Druck von 0,1 bis 1,5 MPa abs oder darüber durchgeführt werden. Geeignete Verfahrensweisen sind etwa die Durchleitung des gasförmigen, gegebenenfalls abgekühlten Reaktoraustrags durch einen mit Absorptionsflüssigkeit gefüllten Behälter oder das Versprühen der Absorptionsflüssigkeit im Gasstrom. Entsprechende Methoden zum Auswaschen von Gasströmen sind dem Fachmann bekannt.

Beim erfindungsgemäßen Verfahren führt man mindestens einen Teil des nicht-umgesetzten n-Butans zur Reaktor-Einheit zurück. Bevorzugt führt man mindestens 40%, besonders bevorzugt 40 bis 80% und ganz besonders bevorzugt 50 bis 75% zur Reaktor-Einheit zurück. Die Rückführung erfolgt indirekt oder direkt unter Einsatz des durch die Abtrennung des gebildeten nnaieinsäureanhydrids entstandenen Gasstroms, welcher nicht-umgesetztes n-Butan und Wasser enthält.

Bei der indirekten Rückführung wird das n-Butan in einer nahezu beliebigen Art und Weise abgetrennt und somit in angereicherter Form zur Reaktor-Einheit zurückgeführt. Ein mögliches Verfahren zur Abtrennung des n-Butans ist die Kondensation, wie beispielsweise in EP-A 0 029 317 beschrieben. Ein weiteres und bevorzugtes Verfahren zur Abtrennung des n-Butans ist die Adsorption an einem geeigneten Adsorptionsmittel mit nachfolgender Desorption, wie beispielsweise in US 5,532,284, US 5,646,304, US 5,726,327 und US 6,002,019 beschrieben.

Bei der direkten Rückführung wird der durch die Abtrennung des gebildeten Maleinsäureanhydrids entstandene Gasstrom in entsprechender Menge zur Reaktor-Einheit zurückgeführt. Dabei ist es je nach den gewünschten Reaktionsbedingungen möglich, den Wassergehalt des rückzuführenden Gasstroms zu erniedrigen, zu belassen oder zu erhöhen. Um eine Aufpegelung unerwünschter Einsatzstoffe, Nebenprodukte und Inertgase zu vermeiden, wird eine entsprechende Menge des genannten Gasstroms ausgeschleust. In der Regel wird dieser Gasstrom der thermischen Verwertung zugeführt.

Bevorzugt ist beim erfindungsgemäßen Verfahren die Abtrennung von Maleinsäureanhydrid aus dem von der Reaktor-Einheit abgeführten Gasstrom und das Zurückführen von mindestens einem Teil des an Maleinsäureanhydrid abgereicherten Gasstroms zur Reaktor-Einheit.

In einer bevorzugten Ausführungsform des erfindungsgemäßen Verfahrens führt man die heterogenkatalytische Gasphasenoxidation von n-Butan in einem Rohrbündelreaktor durch. n-Butan, Luft, rückgeführtes, n-Bütan-haltige Gas und Tri-(C$_1$- bis C$_4$-alkyl)-phosphat werden in entsprechender Menge dem Rohrbündelreaktor kontinuierlich zugeführt. Die n-Butan-Konzentration im Reaktor-Eingangsstrom liegt im Bereich von 1 bis 1,5 Vol.%, die Sauerstoff-Konzentration im Bereich von 10 bis 18 Vol.-%. Der Druck am Eingang des Rohrbündelreaktors liegt im Bereich von 0,4 bis 1 MPa abs. Im Rohrbündelreaktor wird am Vanadium, Phosphor und Sauerstoff enthaltenden Katalysator n-Butan bei einer Temperatur im Bereich von 380 bis 460°C zu Maleinsäureanhydrid umgesetzt. Der aus dem Rohrbündelreaktor abgeführte Gasstrom wird einer Absorber-Einheit zugeführt, in der das gebildete Maleinsäureanhydrid durch ein geeignetes Absorptionsmittel ausgewaschen wird. 40 bis 80% des verbleibenden, das nicht-umgesetzte n-Butan und Wasser enthaltenden Gasstroms werden zum Rohrbündelreaktor zurückgeführt. Der restliche Teile des Gasstroms wird in einem sogenannten Incinerator unter Gewinnung von thermischer Energie verbrannt.

Das erfindungsgemäße Verfahren zur Herstellung.von Maleinsäureanhydrid ermöglicht eine in Bezug auf das Durchgehen der Reaktion sicherheitstechnisch unproblematische Reaktionsführung mit einem entsprechendem Sicherheitsabstand zum Explosionsbereich. Das erfindungsgemäße Verfahren ermöglicht ferner eine hohe Ausnutzung des eingesetzten n-Butans durch Rückführung von mindestens einem Teil des nicht-umgesetzten n-Butans. Es ist in einfacher Art und Weise durchzuführen und ermöglicht einen hohen n-Butan-Umsatz, eine hohe Selektivität zu sowie eine hohe Ausbeute an Maleinsäureanhydrid und daher eine hohe Raum-Zeit-Ausbeute. In der bevorzugten Form erlaubt das erfindungsgemäße Verfahren des Weiteren den Einsatz von gut zugänglicher Luft als Sauerstoff enthaltendes Gas. Weiterhin erlaubt das erfindungsgemäße Verfahren in einer weiteren bevorzugten Form die Rückführung von nicht-umgesetzten n-Butan ohne den Einsatz einer technisch aufwändigen n-Butan-Adsorptionseinheit.

Definitionen

Die in den Beispielen verwendeten Größen sind, falls nicht anders erwähnt, wie folgt definiert:

$$\text{Raum/Zeit-Ausbeute} = \frac{m_{Maleinsäureanhydrid}}{V_{Katalysator} \cdot t}$$

$$\text{Belastung} = \frac{V_{Kohlenwasserstoff}}{V_{Katalysator} \cdot t}$$

$$\text{GHSV (gaseous hourly space velocity)} = \frac{V_{Gas}}{V_{Katalysator} \cdot t}$$

$$\text{Umsatz } U_{n\text{-Butan, per pass}} = \frac{\dot{n}_{n-Butan,in} - \dot{n}_{n-Butan,out}}{\dot{n}_{n-Butan,in}}$$

$$\text{Selektivität } S_{Maleinsäureanhydrid, per pass} = \frac{\dot{n}_{Maleinsäureanhydrid,out}}{\dot{n}_{n-Butan,in} - \dot{n}_{n-Butan,out}}$$

$$\text{Ausbeute } A_{Maleinsäureanhydrid, per pass} = U_{n-Butan, per pass} \cdot S_{Maleinsäureanhydrid, per pass}$$

$$\text{Selektivität } S_{\text{Acrylsäure, per pass}} = \frac{\dot{n}_{Acrylsäure,out}}{\dot{n}_{n-Bu\,tan,in} - \dot{n}_{n-Bu\,tan,out}}$$

$$\text{Selektivität } S_{\text{Essigsäure, per pass}} = \frac{\dot{n}_{Essigsäure,out}}{\dot{n}_{n-Bu\,tan,in} - \dot{n}_{n-Bu\,tan,out}}$$

$$\dot{n}_{n-Bu\,tan,in} = \dot{n}_{n-Bu\,tan,fresh} + \dot{n}_{n-Bu\,tan,recycle}$$

$$\text{Ausbeute } A_{\text{Maleinsäureanhydrid, overall}} = A_{Maleinsäureanhydrid,\,per\,pass} \cdot \frac{\dot{n}_{n-Bu\,tan,in}}{\dot{n}_{n-Bu\,tan,fresh}}$$

$$\text{Rückführrate} = \frac{\dot{n}_{n-Bu\,tan,recycle}}{(\dot{n}_{n-Bu\,tan,recycle} + \dot{n}_{n-Bu\,tan,purge})}$$

| | |
|---|---|
| $m_{\text{Maleinsäureanhydrid}}$ | Masse an produziertem Maleinsäureanhydrid [g] |
| $V_{\text{Katalysator}}$ | Gesamtvolumen, in dem sich während der Durchführung der Umsetzung der Katalysator befindet, inklusive Volumen zwischen den Katalysatorpartikeln [l]. Beim Einsatz eines Rohrbündelreaktors entspricht $V_{\text{Katalysator}}$ dem Schüttvolumen des Katalysator, summiert über alle Reaktionszonen. Beim Einsatz eines Wirbelbettreaktors entspricht $V_{\text{Katalysator}}$ dem Volumen des nicht aufgewirbelten Katalysators. |
| $t$ | Zeiteinheit [h] |
| $V_{\text{Kohlenwasserstoff}}$ | auf 0°C und 0,1013 MPa normiertes Volumen des Kohlenwasserstoffs in der Gasphase am Reaktoreingang [Nl] (Rechnerische Größe. Liegt ein Kohlenwasserstoff unter diesen Bedingungen in der Flüssigphase vor, so wird über das ideale Gasgesetz das hypothetische Gasvolumen berechnet.) |
| $V_{\text{Gas}}$ | auf 0°C und 0,1013 MPa normiertes Volumen der gesamten Gasmenge am Reaktoreingang [Nl] |
| $U_{\text{n-Butan, per pass}}$ | Umsatz an Kohlenwasserstoffen pro Reaktordurchgang |
| $S_{\text{Maleinsäureanhydrid, per pass}}$ | Selektivität zu Maleinsäureanhydrid pro Reaktordurchgang |
| $A_{\text{Maleinsäureanhydrid, per pass}}$ | Ausbeute an Maleinsäureanhydrid pro Reaktordurchgang |
| $S_{\text{Acrylsäure, per pass}}$ | Selektivität zu Acrylsäure pro Reaktordurchgang |
| $S_{\text{Essigsäure, per pass}}$ | Selektivität zu Essigsäure pro Reaktordurchgang |
| $A_{\text{Maleinsäureanhydrid, overall}}$ | Gesamtausbeute an Maleinsäureanhydrid im System |
| $\dot{n}_{n-Bu\,tan,in}$ | n-Butan-Stoffmengenstrom am Reaktoreingang [mol/h] |
| $\dot{n}_{n-Bu\,tan,out}$ | n-Butan-Stoffmengenstrom am Reaktorausgang [mol/h] |
| $\dot{n}_{n-Bu\,tan,fresh}$ | n-Butan-Stoffmengenstrom, welcher dem System von außen frisch zugeführt wird [mol/h] |
| $\dot{n}_{n-Bu\,tan,recycle}$ | n-Butan-Stoffmengenstrom, welcher über das Kreisgas zurückgeführt wird [mol/h] |
| $\dot{n}_{n-Bu\,tan,parge}$ | n-Butan-Stoffmengenstrom, welcher aus dem Kreisgas als Abgas ausgeschleust wird [mol/h] |
| $\dot{n}_{\text{Maleinsäureanhydrid,out}}$ | Maleinsäureanhydrid-Stoffmengenstrom am Reaktorausgang [mol/h] |

| Rückführrate | Rückführrate des n-Butans |
|---|---|
| $T_{SB}$ | Mittlere Salzbad-Temperatur in der Reaktionszone. Diese entspricht dem Mittelwert aus der Temperatur der dem Reaktor zugeführten Salzschmelze und der dem Reaktor abgeführten Salzschmelze. |

Beispiele

Bestimmung der mittleren Oxidationsstufe des Vanadiums

Die Bestimmung der mittleren Oxidationsstufe des Vanadiums erfolgte über potentiometrische Titration. Zur Bestimmung werden jeweils 200 bis 300 mg der Probe unter Argonatmosphäre in eine Mischung aus 15 mL 50%-iger Schwefelsäure und 5 mL 85%-iger Phosphorsäure gegeben und unter Erwärmen gelöst. Die Lösung wird anschließend in ein Titrationsgefäß, welches mit zwei Pt-Elektroden ausgestattet ist, überführt. Die Titrationen werden jeweils bei 80˚C durchgeführt. Zuerst erfolgt eine Titration mit 0,1 molarer Kaliumpermanganat-Lösung. Werden zwei Stufen in der potentiometrischen Kurve erhalten, so lag das Vanadium in einer mittleren Oxidationsstufe von +3 bis kleiner +4 vor. Wird nur eine Stufe erhalten, so lag das Vanadium in einer Oxidationsstufe von +4 bis kleiner +5 vor.

Bei dem erstgenannten Fall (zwei Stufen / $+3 \leq V_{ox} < +4$) enthält die Lösung kein $V^{5+}$, das heißt das gesamte Vanadium wurde titrimetrisch erfaßt. Über den Verbrauch der 0,1 molaren Kaliumpermanganat-Lösung und der Lage der beiden Stufen wird die Menge an $V^{3+}$ und $V^{4+}$ berechnet. Der gewichtete Mittelwert ergibt dann die mittlere Oxidationsstufe.

Bei dem zweitgenannten Fall (eine Stufe / $+4 \leq V_{ox} < +5$) kann aus dem Verbrauch der 0,1 molaren Kaliumpermanganat-Lösung die Menge an $V^{4+}$ berechnet werden. Durch anschließende Reduktion des gesamten $V^{5+}$ der erhaltenen Lösung mit einer 0,1 molaren Ammonium-eisen(II)-sulfat-Lösung und erneute Oxidation mit 0,1 molarer Kaliumpermanganat-Lösung kann die Gesamtmenge an Vanadium berechnet werden. Die Differenz zwischen der Gesamtmenge an Vanadium und der Menge an $V^{4+}$ ergibt die ursprünglich vorhandene Menge an $V^{5+}$. Der gewichtete Mittelwert ergibt dann die mittlere Oxidationsstufe.

Bestimmung der Seitendruckfestigkeit der Hohlzylinder

Zur Bestimmung der Seitendruckfestigkeit wurden in nacheinander folgenden Messungen die Hohlzylinder mit der gerundeten Seitenfläche jeweils auf die plane Metall-Auflageplatte einer entsprechenden Meßeinrichtung gestellt. Die beiden planparallelen Deckelflächen befanden sich somit in vertikaler Richtung. Nun wurde ein planer Metall-Stempel von oben mit einer Vorschubgeschwindigkeit von 1,6 mm/min auf den Hohlzylinder zugefahren und der zeitliche Verlauf der Krafteinwirkung auf den Hohlzylinder bis zu dessen Bruch aufgezeichnet. Die Seitendruckfestigkeit des einzelnen Hohlzylinders entspricht der maximal eingewirkten Kraft.

Zur Bestimmung der Seitendruckfestigkeit wurden unter Mittelwertbildung jeweils 30 Einzelmessungen durchgeführt.

Versuchsanlage 1 (Single Pass)

Die Versuchsanlage 1 war ausgestattet mit einer Zufuhr-Einheit und einem Reaktor rohr. Der Ersatz eines Rohrbündelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" (Single Pass-Fahrweise) betrieben.

Der Kohlenwasserstoff wurde mengengeregelt in flüssiger Form über eine Pumpe zugegeben. Als Sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs betrug 6,5 m, der Innendurchmesser 22,3 mm. Innerhalb des Reaktorrohres befand sich in einem Schutzrohr mit 6 mm Außendurchmesser ein Multi-Thermoelement mit 20 Temperaturmeßstellen. Die Temperierung des Reaktors erfolgte durch einen regelbaren Wärmeträgerkreislauf. Als Wärmeträgermedium wurde eine Salzschmelze eingesetzt.

Das Reaktorrohr wurde von dem Reaktionsgasgemisch von oben nach unten durchströmt. Die oberen 0,2 m des 6,5 m langen Reaktorrohres blieben ungefüllt. Als Nächstes folgte eine 0,3 m lange Vorheizzone, welche mit Steatitformkörpem als Inertmaterial gefüllt war. Im Anschluß an die Vorheizzone folgte die Katalysatorschüttung, welche insgesamt 2180 mL Katalysator enthielt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Versuchsanlage 2 (Kreisgas)

Bei der Versuchsanlage 2 handelte es sich um Versuchsanlage 1, ergänzt um eine Absorbereinheit und eine Kreisgasrückführung. Die Unterschiede zur Versuchsanlgae 1 sind im Folgenden zusammengefasst:

- Kreisgasfahrweise anstatt "gerader Durchgang".
- Katalysatorschüttung mit 2176 mL (anstatt 2180 mL).
- Zuführung des gasförmigen Reaktoraustrags zu einer mit Wasser als Lösungsmittel betriebenen Waschkolonne, um die löslichen organischen Oxidationsprodukte, wie beispielsweise Maleinsäureanhydrid, Acrylsäure und Essigsäure zu entfernen.
- Ausschleusung eines Teils des verbleibenden Gasstroms als Abgas und mengengeregelte Rückführung des anderen Teils als Kreisgasstrom. Der Kreisgasstrom enthielt überwiegend Sauerstoff, Stickstoff, Kohlenmonoxid, Kohlendioxid und nicht umgesetztes n-Butan.

Herstellung des Katalysators 1

In einem mit Stickstoff inertisierten, über Druckwasser außenbeheizbaren 8 $m^3$-Stahl/Email-Rührkessel mit Strombrechem wurden 6,1 $m^3$ Isobutanol vorgelegt. Nach Inbetriebnahme des dreistufigen Impellerrührers wurde das Isobutanol unter Rückfluß auf 90˚C aufgeheizt. Bei dieser Temperatur wurde nun über die Förderschnecke mit der Zugabe von 736 kg Vanadiumpentoxid begonnen. Nachdem nach ca. 20 Minuten etwa 2/3 der gewünschten Menge an Vanadiumpentoxid zugegeben wurden, wurde bei weiterer Zugabe an Vanadiumpentoxid mit der Einpumpung von 900 kg 105%-iger Phosphorsäure begonnen. Zur Reinigung der Pumpe wurden weitere 0,2 $m^3$ Isobutanol nachgepumpt. Anschließend wurde das Reaktionsgemisch unter Rückfluß auf etwa 100 bis 108˚C erhitzt und unter diesen Bedingungen 14 Stunden belassen. Im Anschluß daran wurde die heiße Suspension in eine zuvor mit Stickstoff inertisierte und beheizte Druckfilternutsche abgelassen und bei einer Temperatur von etwa 100˚C bei einem Druck oberhalb der Filternutsche von bis zu 0,35 MPa abs abfiltriert. Der Nutschkucken wurde durch stetiges Einleiten von Stickstoff bei 100˚C und unter Rühren mit einem mittig angeordneten, in der Höhe verstellbaren Rührer innerhalb von etwa einer Stunde trockengeblasen. Nach dem Trockenblasen wurde auf ca. 155˚C aufgeheizt und auf einen Druck von 15 kPa abs (150 mbar abs) evakuiert. Die Trocknung wurde bis zu einem Rest-Isobutanolgehalt von < 2 Gew.-% im getrockneten Katalysator-Precursor durchgeführt.

Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln behandelt. Die Drehzahl des Drehrohres betrug 0,4 U/min. Das Pulver wurde in einer Menge von 60 kg/h in das Drehrohr gefördert. Die Luftzufuhr betrug 100 $m^3$/h. Die direkt an der Außenseite des Drehrohrs gemessenen Temperaturen der fünf gleichlangen Heizzonen betrugen 250˚C, 300˚C, 340˚C, 340˚C und 340˚C. Nach dem Abkühlen auf Raumtemperatur wurde der VPO-Precursor mit 1 Gew.-% Graphit innig vermischt und in einem Walzen-Kompaktor kompaktiert. Das Feingut im Kompaktat mit einer Partikelgröße < 400 $\mu$m wurde abgesiebt und erneut der Kompaktierung zugeführt. Das Grobgut mit einer Partikelgröße ≥ 400 $\mu$m wurde mit weiteren 2 Gew.-% Graphit vermischt und in einer Tablettiermaschine zu 5x3x2,5 mm Hohlzylindern (äußerer Durchmesser x Höhe x Durchmesser des inneren Lochs) mit einer Seitendruckfestigkeit von 11 N tablettiert. Um die erforderliche Menge an Katalysatorprecursor zu erhalten, wurden mehrere Ansätze durchgeführt.

Etwa 2,7 t der erhaltenen 5x3x2,5 mm Hohlzylindern wurden in einer Schütthöhe von 9 bis 10 cm kontinuierlich auf ein gasdurchlässiges Förderband einer Bandkalziniervorrichtung aus zwei hintereinandergeschalteten, identischen Bandkalzinierapparaten mit insgesamt acht Kalzinierzonen gegeben. Die ersten 1,4 t wurden zur einmaligen Einstellung der Betriebsparameter der Bandkalziniervorrichtung verwendet. Da sie kein einheitliches Material darstellten, wurden sie im Folgenden nicht weiter betrachtet.

Die Bandkalziniervorrichtung wurde bei Atmosphärendruck betrieben. Zwischen den Kalzinierzonen 4 und 5 befand sich eine umkapselte Übergangszone. Jede der acht Kalzinierzonen umfasste zur Erzeugung einer Gaszirkulation jeweils einen Ventilator. Jede der acht Kalzinierzonen wurde mit der gewünschten Menge an gewünschtem Frischgas versorgt. Zur Erhaltung des gewünschten Atmosphärendrucks wurde eine entsprechende Gasmenge abgeführt. Das Volumen des pro Zeiteinheit in jeder Kalzinierungszone zirkulierenden Gases war größer als das Volumen des pro Zeiteinheit zu- oder abgeführten Gases. Zwischen zwei aufeinanderfolgenden Kalzinierungszonen befand sich zur Verringerung des Gasaustausches jeweils eine Trennwand, welche im Bereich des Stromes des Katalysatorprecursors offen war. Die Länge jeder Kalzinierzone betrug 1,45 m. Die Geschwindigkeit des Förderbandes wurde entsprechend der gewünschten Verweilzeit von etwa 2 Stunden pro Kalzinierzone eingestellt. Die einzelnen Zonen wurden wie in Tabelle 1 dargestellt betrieben:

Tabelle 1: Parameter zum Betrieb der Bandkalziniervorrichtung.

| Zone | Temperatur | zugeführtes Frischgas |
|---|---|---|
| Kalzinierzone 1 | Aufheizen auf 250˚C | Luft |
| Kalzinierzone 2 | Halten bei 250˚C | Luft |
| Kalzinierzone 3 | Halten bei 250˚C | Luft |
| Kalzinierzone 4 | Aufheizen auf 310˚C | Luft |
| Übergangszone | Abkühlen auf 200˚C | Luft |
| Kalzinierzone 5 | Aufheizen auf 425˚C | $N_2$ |
| Kalzinierzone 6 | Halten bei 425˚C | $N_2/H_2O$-Dampf (1:1) |
| Kalzinierzone 7 | Halten bei 425˚C | $N_2/H_2O$-Dampf (1:1) |
| Kalzinierzone 8 | Abkühlen auf Raumtemperatur | $N_2$ |

Auf diese Weise wurden ca. 1,3 t fertiger Katalysator 1 kontinuierlich hergestellt. Eine repräsentative Durchschnittsprobe dieses Katalysators wies folgende Daten auf:

* mittlere Oxidationsstufe des Vanadiums ($V_{ox}$): 4,15
* Seitendruckfestigkeit (SDF): 9,4 N Herstellung von Katalysator 2

Die Herstellung des getrockneten Katalysator-Precursor-Pulvers erfolgte wie bei Katalysator 1 beschrieben.
Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln behandelt. Die Drehzahl des Drehrohres betrug 0,4 U/min. Das Pulver wurde in einer Menge von 60 kg/h in das Drehrohr gefördert. Die Luftzufuhr betrug 100 m3/h. Die direkt an der Außenseite des Drehrohrs gemessenen Temperaturen der fünf gleichlangen Heizzonen betrugen 250˚C, 300˚C, 340˚C, 340˚C und 340˚C. Nach dem Abkühlen auf Raumtemperatur wurde der VPO-Precursor mit 1 Gew.-% Graphit innig vermischt und in einem Walzen-Kompaktor kompaktiert. Das Feingut im Kompaktat mit einer Partikelgröße < 400 $\mu$m wurde abgesiebt und erneut der Kompaktierung zugeführt. Das Grobgut mit einer Partikelgröße ≥ 400 $\mu$m wurde mit weiteren 2 Gew.-% Graphit vermischt und in einer Tablettiermaschine zu 5,5x3x3 mm Hohlzylindern (äußerer Durchmesser x Höhe x Durchmesser des inneren Lochs) mit einer Seitendruckfestigkeit von 10 N tablettiert. Um die erforderliche Menge an Katalysatorprecursor zu erhalten, wurden mehrere Ansätze durchgeführt.
Die 5,5x3x3 mm Hohlzylinder wurden in einer Schütthöhe von 9 bis 10 cm kontinuierlich auf ein gasdurchlässiges Förderband einer Bandkalziniervorrichtung aus zwei hintereinandergeschalteten, identischen Bandkalzinierapparaten mit insgesamt acht Kalzinierzonen gegeben. Die Kalzinierung erfolgte wie bei Katalysator 1 beschrieben.
Es wurde eine repräsentative Probe genommen. Diese wies folgende Daten auf:

* mittlere Oxidationsstufe des Vanadiums (Vox): 4,14
* Seitendruckfestigkeit (SDF): 8 N

Vorbemerkung zu den Beispielen 1 bis 11

Die Beispiele 1 bis 11 wurden in der beschriebenen Versuchanlage 1 unter Einsatz des beschriebenen Katalysators 1 durchgeführt. Die genannten Meßergebnisse wurden nach einer Katalysatorlaufzeit von etwa 300 Stunden ermittelt. Unter den vorgegebenen Parametern wurde durch Einstellung der mittleren Salzbad-Temperatur $T_{SB}$ jeweils ein Umsatz $U_{n\text{-Butan, per pass}}$ von etwa 80% eingestellt, so dass die einzelnen Beispiele untereinander vergleichbar sind.
Auch wenn die Versuchsanlage 1 im "geraden Durchgang" (Single Pass-Fahrvveise) betrieben wurde, wurde die Zusammensetzung des Reaktor-Eingangsstroms und die Verfahrensparameter im Reaktor bezüglich der n-Butan-Konzentration, der Sauerstoff-Konzentration und des Druckes analog einer entsprechenden Fahrweise mit n-Butan-Rückführung (Recycle Pass-Fahrweise) eingestellt. Die Beispiele sind daher in Bezug auf den Betrieb des Reaktors und die Performance-Daten wie insbesondere Umsatz, Selektivität, Ausbeute und Raum/Zeit-Ausbeute auch für eine entsprechende Fahrweise mit n-Butan-Rückführung (Recycle Pass-Fahrweise) repräsentativ und übertragbar.

Beispiele 1 bis 5 (Vergleichsbeispiele)

Bei diesen Beispielen wurde die Sauerstoff-Konzentration bei konstantem Reaktor-Eingangsdruck von 0,33 MPa abs und konstanter n-Butan-Konzentration im Reaktor Eingangsstrom von 2,0 Vol.-% von anfänglich 19,9 Vol.-% auf 12,4 Vol.-% variiert. Die Variation erfolgte durch partielle Substitution von Luft durch Stickstoff.

Die in Tabelle 2 wiedergegebenen Ergebnisse zeigen, dass bei Verringerung der Sauerstoff-Konzentration von 19,9 Vol.-% auf 12,4 Vol.-% und damit bei entsprechender Verringerung des Sauerstoff-Partialdrucks die Maleinsäureanhydrid-Selektivität um 3,9% und die Maleinsäureanhydrid-Ausbeute um 2,4% sinkt. Die Raum/Zeit-Ausbeute verringerte sich um 4,2 g/lh. Gleichzeitig steigt die für den angestrebten n-Butan-Umsatz von etwa 80% erforderliche mittlere Salzbad-Temperatur um 15°C von 404 auf 419°C an, was zu einer deutlich schnelleren Desaktivierung des Katalysators und somit einer signifikant kürzeren Laufzeit führt.

Beispiele 6 bis 8 (Vergleichsbeispiele)

Bei diesen Beispielen wurde die Sauerstoff-Konzentration bei konstantem Sauerstoff-Partialdruck von 0,066 MPa und konstanter n-Butan-Konzentration im Reaktor-Eingangsstrom von 2,0 Vol.-% von anfänglich 19,9 Vol.-% auf 15,0 Vol.-% variiert. Damit verbunden war eine Anhebung des Reaktor-Eingangsdrucks von 0,33 auf 0,44 MPa abs. Die Variation erfolgte durch partielle Substitution von Luft durch Stickstoff.

Die in Tabelle 3 wiedergegebenen Ergebnisse zeigen, dass bei Verringerung der Sauerstoff Konzentration von 19,9 Vol.-% auf 15,0 Vol.-% bei konstantem Sauerstoff-Partialdruck und damit bei entsprechender Erhöhung des Reaktor-Eingangsdrucks die Maleinsäureanhydrid-Selektivität um 1,3% und die Maleinsäureanhydrid-Ausbeute um 0,9% sinkt, wenn die n-Butan-Konzentration im Reaktor-Eingangsstrom 2,0 Vol.-% beträgt. Die Raum/Zeit-Ausbeute verringerte sich um 1,6 g/lh.

Beispiele 9 (Vergleichsbeispiel) und Beispiele 10 und 11

Bei diesen Beispielen wurde die Sauerstoff-Konzentration bei konstantem Sauerstoff-Partialdruck von 0,066 MPa von anfänglich 19,9 Vol.-% auf 12,6 Vol.-% und die n-Butan-Konzentration im Reaktor-Eingangsstrom von anfänglich 2,0 Vol.-% auf 1,2 Vol.-% variiert. Damit verbunden war eine Anhebung des Reaktor-Eingangsdrucks von 0,33 auf 0,52 MPa abs. Die Variation erfolgte durch partielle Substitution von Luft durch Stickstoff sowie durch eine Verringerung der zugeführten n-Butan-Menge.

Die in Tabelle 4 wiedergegebenen Ergebnisse zeigen, dass die Sauerstoff-Konzentration von 19,9 Vol.-% auf 12,6 Vol.-% bei konstantem Sauerstoff-Partialdruck und damit bei entsprechender Erhöhung des Reaktor-Eingangsdrucks ohne nennenswerten Ausbeuteverlust an Maleinsäureanhydrid und ohne Erhöhung der Bildung der Nebenkomponenten Acrylsäure und Essigsäure verringert werden kann, wenn die n-Butan-Konzentration im Reaktor-Eingangsstrom gleichzeitig von 2,0 Vol.-% auf 1,2 Vol.-% erniedrigt wird. Die aus den Versuchsdaten berechnete geringe Abnahme der Maleinsäureanhydrid-Ausbeute von 0,2% wird von dem enormen Vorteil, dass im Gegensatz zu einer Sauerstoff-Konzentration von 19,9 Vol.-%, bei der die technisch aufwändige und teuere Zugabe von reinem Sauerstoff erforderlich ist, bei einer Sauerstoff-Konzentration von 15,2 Vol.-% und darunter Luft als Sauerstoff enthaltendes Gas zugegeben werden kann, überkompensiert. Das erfindungsgemäße Verfahren weist somit trotz einer eventuell geringfügig niedrigeren Maleinsäureanhydrid-Ausbeute in seiner Gesamtheit aufgrund des nun möglichen Einsatzes von Luft einen deutlichen Vorteil gegenüber den Verfahren nach dem Stand der Technik auf.

Vorbemerkung zu den Beispielen 12 bis 25

Die Beispiele 12 bis 25 wurden in der beschriebenen Versuchanlage 2 unter Einsatz des beschriebenen Katalysators 2 durchgeführt. Die genannten Meßergebnisse wurden nach einer Katalysatorlaufzeit von etwa 300 Stunden ermittelt. Unter den vorgegebenen Parametern wurde durch Einstellung der mittleren Salzbad-Temperatur TSB jeweils ein Umsatz $Un_{Butan, per pass}$ von etwa 85% eingestellt, so dass die einzelnen Beispiele untereinander vergleichbar sind.

Bei diesen Beispielen wurde die Menge an rückgeführtem Abgas bei konstanter n-Butan-Konzentration und konstanter GHSV variiert. Zum Vergleich wurden die Beispiele 12, 13, 19 und 20 ohne Rückführung durchgeführt.

Die in Tabelle 5 wiedergegebenen Ergebnisse zeigen, dass durch Rückführung des n-Butans eine Steigerung der Maleinsäureanhydrid-Ausbeute $A_{Maleinsäureanhydrid, overall}$ von 58% auf 63,1 % erzielt wird. Zudem kann die Salzbad-Temperatur von 416°C auf bis zu 394°C abgesenkt werden. Die Menge an Triethylphosphat kann beim erfindungsgemäßen Verfahren zurückgenommen werden und hat eine weitere Verbesserung der Wirtschaftlichkeit des Verfahrens zur Folge. Die Raum/Zeit-Ausbeute verringert sich bei konstanter n-Butan-Belastung nur geringfügig.

Die in Tabelle 6 wiedergegebenen Ergebnisse zeigen, dass durch Rückführung des n-Büiäns nach dem erfindungsgemäßen Verfahren zudem die Raum/Zeit-Ausbeute auf bis zu 130,5 g/lh gesteigert werden kann, da auch bei höheren n-Butan-Belastungen noch ein sicherer Betrieb des Reaktors möglich ist. Zwar kann durch Änderung der Betriebsparameter des single pass-Verfahrens auf die Parameter der Kreisgasfahrweise ein stabiler Betriebspunkt unter Erzielung einer vergleichbaren Raum/Zeit-Ausbeute angefahren werden, jedoch ist die Maleinsäureanhydrid-Ausbeute $A_{Maleinsäureanhydrid, overall}$ beim erfindungsgemäßen Verfahren um 5,4 abs-% bzw. um 9,6 rel-% höher als beim single pass-Verfahren.

Tabelle 2: Variation der Sauerstoff-Konzentration bei konstantem Reaktor-Eingangsdruck von 0,33 MPa abs und konstanter n-Butan-Konzentration im Reaktor-Eingangsstrom von 2,0 Vol.%.

| | Beispiel 1 | Beispiel 2 | Beispiel 3 | Beispiel 4 | Beispiel 5 |
|---|---|---|---|---|---|
| Reaktor-Eingangsdruck [MPa abs] | 0,33 | 0,33 | 0,33 | 0,33 | 0,33 |
| Sauerstoff-Konzentration [Vol.-%] | 19,9 | 16,4 | 15,3 | 13,7 | 12,4 |
| n-Butan-Konzentration [Vol.-%] | 2,0 | 2,0 | 2,0 | 2,0 | 2,0 |
| $U_{n-Butan, per pass}$ [%] | 79,4 | 80,3 | 80,3 | 80,4 | 80,4 |
| $T_{SB}$ [°C] | 404 | 412 | 412 | 416 | 419 |
| $A_{Maleinsäureanhydrid, per pass}$ [%] | 56,5 | 55,4 | 54,9 | 54,6 | 54,1 |
| $S_{Maleinsäureanhydrld, per pass}$ [%] | 71,2 | 69,0 | 68,4 | 68,0 | 67,3 |
| $S_{Acrylsäure, per pass}$ + $S_{Essigsäure, per pass}$ [%] | 3,3 | 2,9 | 2,9 | 2,8 | 2,6 |
| Raum/Zeit-Ausbeute [g/lh] | 98,9 | 97,0 | 96,1 | 95,6 | 94,7 |
| Während der gesamten Versuchsreihe wurden folgende Parameter konstantgehalten:<br>* GHSV = 2000 Nl/l$_{Katalysator}$·h<br>* Konzentration an Triethylphosphat (TEP) = 2 Vol.-ppm<br>* Konzentration an Wasserdampf = 3 Vol.-% | | | | | |

Tabelle 3: Variation der Sauerstoff-Konzentration bei konstantem Sauerstoff-Partialdruck von 0,066 MPa und konstanter n-Butan-Konzentration im Reaktor-Eingangsstrom von 2,0 Vol.%.

| | Beispiel 6 | Beispiel 7 | Beispiel 8 |
|---|---|---|---|
| Reaktor-Eingangsdruck [MPa abs] | 0,33 | 0,42 | 0,44 |
| Sauerstoff-Konzentration [Vol.-%] | 19,9 | 15,4 | 15,0 |
| n-Butan-Konzentration [Vol.-%] | 2,0 | 2,0 | 2,0 |
| $U_{n-Butan, per pass}$ [%] | 79,8 | 80,2 | 80,0 |
| $T_{SB}$ [°C] | 407 | 404 | 404 |
| $A_{Maleinsäureanhydrid, per pass}$ [%] | 55,6 | 55,0 | 54,7 |
| $S_{Maleinsäureanhyddd, per pass}$ [%] | 69,7 | 68,5 | 68,4 |
| $S_{Acrylsäure, per pass}$ + $S_{Essigsäure, per pass}$ [%] | 3,1 | 3,4 | 3,5 |

(fortgesetzt)

|  | Beispiel 6 | Beispiel 7 | Beispiel 8 |
|---|---|---|---|
| Raum/Zeit-Ausbeute [g/lh] | 97,3 | 96,3 | 95,7 |
| Während der gesamten Versuchsreihe wurden folgende Parameter konstantgehalten:<br>* GHSV = 2000 Nl/l$_{Katalysator}$·h<br>* Konzentration an Triethylphosphat (TEP) = 2 Vol.-ppm<br>* Konzentration an Wasserdampf = 3 Vol.-% | | | |

Tabelle 4: Variation der Sauerstoff-Konzentration bei konstantem Sauerstoff-Partialdruck von 0,066 MPa und Variation der n-Butan-Konzentration im Reaktor-Eingangsstrom.

|  | Beispiel 9 | Beispiel 10 | Beispiel 11 |
|---|---|---|---|
| Reaktor-Eingangsdruck [MPa abs] | 0,33 | 0,44 | 0,52 |
| Sauerstoff-Konzentration [Vol.-%] | 19,9 | 15,2 | 12,6 |
| n-Butan-Konzentration [Vol.-%] | 2,0 | 1,5 | 1,2 |
| GHSV [Nl/l$_{Katalysator}$ · h] | 2000 | 2600 | 3300 |
| U$_{n\text{-}Butan, per pass}$ [%] | 80,2 | 80,0 | 80,3 |
| T$_{SB}$ [°C] | 407 | 408 | 412 |
| A$_{Maleinsäureanhydrid, per pass}$ [%] | 55,7 | 55,6 | 55,5 |
| S$_{Maleinsäureanhydrid, per pass}$ [%] | 69,4 | 69,5 | 69,1 |
| S$_{Acrylsäure, per pass}$ + S$_{Essigsäure, per pass}$ [%] | 3,1 | 3,2 | 3,1 |
| Raum/Zeit-Ausbeute [g/lh] | 97,5 | 97,3 | 97,1 |
| Während der gesamten Versuchsreihe wurden folgende Parameter konstantgehalten:<br>* Konzentration an Triethylphosphat (TEP) = 2 Vol.-ppm<br>* Konzentration an Wasserdampf = 3 Vol.-% | | | |

Tabelle 5: Variation der Rückführrate bei konstanter n-Butan-Belastung von 44 NL/lh.

|  | Beispiel 12 | Beispiel 13 | Beispiel 14 | Beispiel 15 | Beispiel 16 | Beispiel 17 | Beispiel 18 |
|---|---|---|---|---|---|---|---|
| Rückführrate [%] | 0 | 0 | 38,6 | 54,5 | 65,1 | 74,1 | 79,4 |
| Reaktor-Eingangsdruck [MPa abs] | 0,33 | 0,85 | 0,85 | 0,85 | 0,85 | 0,85 | 0,85 |
| Sauerstoff-Konzentration [Vol.-%] | 19,9 | 19,9 | 17,8 | 16,3 | 14,3 | 11,5 | 8,4 |
| n-Butan-Konzentration [Vol.-%] | 2,0 | 1,0 | 1,0 | 1,0 | 1,0 | 1,0 | 1,0 |
| GHSV [Nl/l$_{Katalysator}$ · h] | 2200 | 4400 | 4400 | 4400 | 4400 | 4400 | 4400 |
| U$_{n\text{-}Bulan, per pass}$ [%] | 84,5 | 84,6 | 84,8 | 84,5 | 84,8 | 84,8 | 85,1 |

(fortgesetzt)

| | Beispiel 12 | Beispiel 13 | Beispiel 14 | Beispiel 15 | Beispiel 16 | Beispiel 17 | Beispiel 18 |
|---|---|---|---|---|---|---|---|
| TSB [˚C] | 416 | 394 | 394 | 396 | 399 | 410 | 420 |
| $A_{Maleinsäureanhydrid, overall}$ [%] | 58,0 | 57,9 | 61,5 | 62,6 | 62,3 | 63,1 | 61,6 |
| Raum/Zeit-Ausbeute [g/lh] | 111,7 | 111,5 | 111,5 | 110,3 | 108,3 | 107,8 | 104,6 |
| TEP [Vol.-ppm] | 2,5 | 1,5 | 1,5 | 1,5 | 1,5 | 1,5 | 1,5 |
| Während der gesamten Versuchsreihe wurden folgende Parameter konstantgehalten: * Konzentration an Wasserdampf = 3 Vol.-% | | | | | | | |

Tabelle 6: Variation der Rückführrate bei konstanter n-Butan-Belastung von 53 NL/lh.

| | Beispiel 19 | Beispiel 20 | Beispiel 21 | Beispiel 22 | Beispiel 23 | Beispiel 24 | Beispiel 25 |
|---|---|---|---|---|---|---|---|
| Rückführrate [%] | 0 | 0 | 39 | 50 | 60 | 64,5 | 79,4 |
| Reaktor-Eingangsdruck [MPa abs] | 0,33 | 0,85 | 0,85 | 0,85 | 0,85 | 0,85 | 0,85 |
| Sauerstoff-Konzentration [Vol.-%] | 19,9 | 19,9 | 17,1 | 15,8 | 14,1 | 13,2 | 8,7 |
| n-Butan-Konzentration [Vol.-%] | 2,4 | 1,2 | 1,2 | 1,2 | 1,2 | 1,2 | 1,2 |
| GHSV [Nl/l$_{Katalysator}$ · h] | 2200 | 4400 | 4400 | 4400 | 4400 | 4400 | 4400 |
| $U_{n-Butan, per pass}$ [%] | runaway | 84,6 | 85 | 84,6 | 85,1 | 85,2 | 84,8 |
| TSB [˚C] | n.b. | 392 | 397 | 399 | 402 | 405 | 419 |
| $A_{Maleinsäureanhydrid,overall}$ [%] | n.b. | 56,1 | 60,0 | 60,7 | 61,5 | 60,8 | 59,2 |
| Raum/Zeit-Ausbeute [g/lh] | n.b. | 129,6 | 130,5 | 129,6 | 129,4 | 127,1 | 121,3 |
| TEP [Vol.-ppm] | 2,5 | 1,5 | 1,5 | 1,5 | 1,5 | 1,5 | 1,5 |
| n.b.: nicht bestimmt Während der gesamten Versuchsreihe wurden folgende Parameter konstantgehalten: * Konzentration an Wasserdampf = 3 Vol.-% | | | | | | | |

**Patentansprüche**

1. Verfahren zur Herstellung von Maleinsäureanhydrid durch heterogenkatalytische Gasphasenoxidation von n-Butan mit Sauerstoff enthaltenden Gasen an einem Vanadium, Phosphor und Sauerstoff enthaltenden Katalysator in einer Reaktor-Einheit bei einer Temperatur im Bereich von 350 bis 500˚C, Abtrennung des gebildeten Maleinsäureanhydrids unter Bildung eines Gasstroms, welcher nicht-umgesetztes n-Butan und Wasser enthält und Rückführung von mindestens einem Teil des nicht-umgesetzten n-Butans zur Reaktor-Einheit, **dadurch gekennzeichnet, dass** man der Reaktor-Einheit einen Eingangsstrom mit einer n-Butan-Konzentration von 0,5 bis 1,5 Vol.-% und einer Sauerstoff-Konzentration von 5 bis 21 Vol.-% zuführt, am Eingang der Reaktor-Einheit einen Druck von 0,4 bis 2 MPa abs einstellt und pro Reaktordurchgang 40 bis 100% des n-Butans aus dem Eingangsstrom umsetzt.

2. Verfahren nach Anspruch 1, **dadurch gekennzeichnet, dass** man der Reaktor-Einheit einen Eingangsstrom mit einer n-Butan-Konzentration von 1 bis 1,5 Vol.-% zuführt.

3. Verfahren nach den Ansprüchen 1 bis 2, **dadurch gekennzeichnet, dass** man der Reaktor-Einheit einen Eingangsstrom mit einer Sauerstoff-Konzentration von 10 bis 18 Vol.-% zuführt.

4. Verfahren nach den Ansprüchen 1 bis 3, **dadurch gekennzeichnet, dass** man der Reaktor-Einheit einen Eingangsstrom mit einem Druck von 0,4 bis 1 MPa abs zuführt.

5. Verfahren nach den Ansprüchen 1 bis 4, **dadurch gekennzeichnet, dass** man über die Menge des Eingangsstroms in der Reaktor-Einheit eine GHSV von 2000 bis 10000 $h^{-1}$, bezogen auf das auf 0˚C und 0,1013 MPa abs normierte Volumen des zugeführten Eingangsstroms und bezogen auf das Schüttvolumen des Katalystors summiert über alle Reaktionszonen, einstellt.

6. Verfahren nach den Ansprüchen 1 bis 5, **dadurch gekennzeichnet, dass** man als Sauerstoff enthaltendes Gas Luft einsetzt.

7. Verfahren nach den Ansprüchen 1 bis 6, **dadurch gekennzeichnet, dass** man die heterogenkatalytische Gasphasenoxidation in Gegenwart einer flüchtigen Phosphorverbindung durchführt.

8. Verfahren nach den Ansprüchen 1 bis 7, **dadurch gekennzeichnet, dass** man als Reaktor-Einheit eine Wirbelbettreaktor-Einheit einsetzt.

9. Verfahren nach den Ansprüchen 1 bis 7, **dadurch gekennzeichnet, dass** man als Reaktor-Einheit eine Rohrbündelreaktor-Einheit mit mindestens einer durch ein Wärmeträgermedium gekühlten Reaktionszone einsetzt.

10. Verfahren nach Anspruch 9, **dadurch gekennzeichnet, dass** man eine Rohrbündelreaktor-Einheit mit mindestens zwei durch ein Wärmeträgermedium gekühlten Reaktionszonen einsetzt.

11. Verfahren nach den Ansprüchen 1 bis 10, **dadurch gekennzeichnet, dass** man mindestens 40 % des nichtumgesetzten n-Butans zur Reaktor-Einheit zurückführt.

12. Verfahren nach Anspruch 11, **dadurch gekennzeichnet, dass** man 40 bis 80 % des nicht-umgesetzten n-Butans zur Reaktor-Einheit zurückführt.

13. Verfahren nach den Ansprüchen 1 bis 12, **dadurch gekennzeichnet, dass** man aus dem von der Reaktor-Einheit abgeführten Gasstrom Maleinsäureanhydrid abtrennt und mindestens einen Teil des an Maleinsäureanhydrid abgereicherten Gasstroms zur Reaktor-Einheit zurückführt.

**Claims**

1. A process for preparing maleic anhydride by oxidizing n-butane in the gas phase under heterogeneous catalysis with oxygen-comprising gases over a vanadium-, phosphorus- and oxygen-containing catalyst in a reactor unit at a temperature in the range from 350 to 500˚C, removing the maleic anhydride formed to form a gas stream which comprises unconverted n-butane and water and recycling at least a portion of the unconverted n-butane to the reactor unit, which comprises feeding to the reactor unit an inlet stream having an n-butane concentration of from 0.5 to 1.5% by volume and an oxygen concentration of from 5 to 21 % by volume, establishing a pressure at the inlet to the reactor unit of from 0.4 to 2 MPa abs, and converting from 40 to 100% of the n-butane from the inlet stream per reactor pass.

2. The process according to claim 1, wherein an inlet stream having an n-butane concentration of from 1 to 1.5% by volume is fed to the reactor unit.

3. The process according to either of claims 1 and 2, wherein an inlet stream having an oxygen concentration of from 10 to 18% by volume is fed to the reactor unit.

4. The process according to any of claims 1 to 3, wherein an inlet stream having a pressure of from 0.4 to 1 MPa abs is fed to the reactor unit.

5. The process according to any of claims 1 to 4, wherein a GHSV of from 2000 to 10 000 $h^{-1}$, based on the volume of the inlet stream fed, normalized to 0˚C and 0.1013 MPa abs, and based on the bed volume of the catalyst summed over all reaction zones, is established in the reactor unit via the flow rate of the inlet stream.

6. The process according to any of claims 1 to 5, wherein the oxygen-comprising gas used is air.

7. The process according to any of claims 1 to 6, wherein the heterogeneously catalyzed gas phase oxidation is carried out in the presence of a volatile phosphorus compound.

8. The process according to any of claims 1 to 7, wherein the reactor unit used is a fluidized bed reactor unit.

9. The process according to any of claims 1 to 7, wherein the reactor unit used is a tube bundle reactor unit having at least one reaction zone cooled by a heat carrier medium.

10. The process according to claim 9, wherein a tube bundle reactor unit is used which has at least two reaction zones cooled by a heat carrier medium.

11. The process according to any of claims 1 to 10, wherein at least 40% of the unconverted n-butane is recycled to the reactor unit.

12. The process according to claim 11, wherein from 40 to 80% of the unconverted n-butane is recycled to the reactor unit.

13. The process according to any of claims 1 to 12, wherein maleic anhydride is removed from the gas stream withdrawn from the reactor unit and at least a portion of the gas stream depleted in maleic anhydride is recycled to the reactor unit.

**Revendications**

1. Procédé pour la préparation d'anhydride maléique par oxydation en phase gazeuse à catalyse hétérogène de n-butane avec des gaz contenant de l'oxygène sur un catalyseur contenant du vanadium, du phosphore et de l'oxygène dans une unité de réacteur à une température située entre 350 et 500 ˚C, séparation de l'anhydride maléique formé avec formation d'un flux de gaz qui contient du n-butane non transformé et de l'eau et retour d'au moins une partie du n-butane non transformé vers l'unité de réacteur, **caractérisé en ce que** l'on amène à l'unité de réacteur un courant d'entrée avec une concentration en n-butane de 0,5 à 1,5 % en volume et une concentration en oxygène de 5 à 21 % en volume, l'on règle à l'entrée de l'unité de réacteur une pression de 0,4 à 2 MPa abs. et l'on transforme, par passage dans le réacteur, 40 à 100 % du n-butane provenant du courant d'entrée.

2. Procédé suivant la revendication 1, **caractérisé en ce que** l'on amène à l'unité de réacteur un courant d'entrée avec une concentration en n-butane de 1 à 1,5 % en vol.

3. Procédé suivant les revendications 1 à 2, **caractérisé en ce que** l'on amène à l'unité de réacteur un courant d'entrée avec une concentration en oxygène de 10 à 18 % en vol.

4. Procédé suivant les revendications 1 à 3, **caractérisé en ce que** l'on amène à l'unité de réacteur un courant d'entrée avec une pression de 0,4 à 1 MPa abs.

5. Procédé suivant les revendications 1 à 4, **caractérisé en ce que** l'on ajuste, via la quantité du courant d'entrée dans l'unité de réacteur, un GHSV de 2000 à 10 000 $h^{-1}$ par rapport au volume normalisé à 0 ˚C et 0,1013 MPa abs. du courant d'entrée amené et par rapport au volume en vrac du catalyseur additionnés sur toutes les zones de réaction.

6. Procédé suivant les revendications 1 à 5, **caractérisé en ce que** l'on met en oeuvre de l'air comme gaz contenant de l'oxygène.

7. Procédé suivant les revendications 1 à 6, **caractérisé en ce que** l'on exécute l'oxydation en phase gazeuse à catalyse hétérogène en présence d'un composé volatil du phosphore.

8. Procédé suivant les revendications 1 à 7, **caractérisé en ce que** l'on met en oeuvre une unité de réacteur à lit fluidisé comme unité de réacteur.

9. Procédé suivant les revendications 1 à 7, **caractérisé en ce que** l'on met en oeuvre comme unité de réacteur une unité de réacteur à faisceau tubulaire avec au moins une zone de réaction refroidie par un agent caloporteur.

10. Procédé suivant la revendication 9, **caractérisé en ce que** l'on met en oeuvre une unité de réacteur à faisceau tubulaire avec au moins deux zones de réaction refroidies par un agent caloporteur.

11. Procédé suivant les revendications 1 à 10, **caractérisé en ce que** l'on retourne au moins 40 % du n-butane non transformé vers l'unité de réacteur.

12. Procédé suivant la revendication 11, **caractérisé en ce que** l'on retourne 40 à 80 % du n-butane non transformé vers l'unité de réacteur.

13. Procédé suivant les revendications 1 à 12, **caractérisé en ce que** l'on sépare l'anhydride maléique du flux de gaz extrait de l'unité de réacteur et l'on retourne au moins une partie du flux de gaz enrichi en anhydride maléique vers l'unité de réacteur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5532284 A
- US 5646304 A
- US 5726327 A
- US 6002019 A
- US 3904652 A
- EP 0029317 A
- US 5011945 A
- EP 0099431 A
- US 4231943 A
- EP 0690040 A
- US 5275996 A
- US 5641722 A
- US 5137860 A
- US 5095125 A
- US 4933312 A
- US 5498731 A
- US 4525471 A
- US 5302566 A
- DE 3429164

- WO 9712674 A
- WO 9526817 A
- US 5296436 A
- US 5158923 A
- US 4795818 A
- EP 0593646 A
- US 5168090 A
- WO 0168626 A
- WO 0168245 A
- WO 0222257 A
- WO 02134387 A
- DE Z10211449 A
- DE Z10211445 A
- DE Z10211447 A
- DE Z10211446 A
- DE Z10235355 A
- US 3474041 A
- WO 9743242 A

### In der Beschreibung aufgeführte Nicht-Patentliteratur

- MALEIC AND FUMARIC ACID - Maleic Anhydride. Ullmann's Encyclopedia of Industrial Chemistry. 1999